# EUROPEAN PATENT APPLICATION

(11) **EP 3 521 443 A1**
(43) Date of publication of application: **07.08.2019**
(21) Application number: 16001462.7
(22) Date of filing: 30.06.2016
(51) Int. Cl.: C12Q 1/26, G01N 33/574

(54) **SALIVA TEST FOR EARLY CANCER DIAGNOSIS**

(30) Priority: 01.07.2015 IT UB20152011
(71) Applicant: Romano, Pietrina, 73018 Squinzano (LE) (IT); Guerrieri, Aurora, 73018 Squinzano (FG) (IT); Romano, Maria Pia, 73018 Squinzano (LE) (IT); Romano, Gianpiero, 73018 Squinzano (LE) (IT)
(72) Inventor: Guerrieri, Aurora, 73018 Squinzano (LE) (IT)
(74) Representative: Russo, Dimitri

(57) **Abstract**

This test is colorimetric rapid, non-invasive, based on quantitative indirect analysis of total L-fucosc in saliva, by means of the intake of molecular iodine (I₂); the salivary concentration of this metilpentose increases from the earliest stages of carcinogenesis.

Method for the early detection of cancer, characterized in that 1ml of saliva reacts with a hydroalcoholic solution of molecular iodine to 5%; this solution shows the increase salivary concentration of L-fucose, by means of the absorption of iodine.

The iodine absorption is due to the reaction between two apolar groups: the methylic residue of L-fucose, (free and bound to glycidic or protein residues) and the molecular iodine. This reaction determines the color change of the sample: the test is positive if the color change occurs (from brown to white) in a specific time interval (<1.5 minutes).

The test can be associated with the different diagnostic prevention methods already in use for several types of cancer.

## Description

### TECHNICAL FIELD

SALIVARY TESTS FOR THE EARLY DETECTION OF CANCER is rapid, non invasive colorimetric test based on quantitative and indirect analysis of total L-fucose in the saliva, by means of the intake of molecular iodine (I2).

The diagnosis of cancer based on two types of interlinked approaches: genetic and metabolic; moreover the structural, functional and energetic glycomics modificatios are fields of study of numerous international trials.

Among the numerous modifications of glycans and glucose metabolism play a central role the Methyl-fucose (L-fucose), the only levorotatory sugar utilized by mammalian systems that increases in persistent hypoxic conditions when activating anaerobic glycolysis and the other energy shunt.

The increase of L-fucose indicates an alteration of the oxidative metabolism of acrobic organisms under various stressing conditions, among which low oxygen levels (hypoxia) and of undifferentiated anaerobic cell survival.

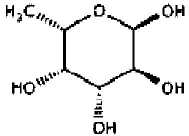

### BACKGROUND ART

L-fucose (6-Deoxy-L-galactose) (C6H12O5) is a hexose deoxy sugar with amphiphilic properties; structural features distinguish Fucose from other six-carbon sugars present in mammals: the lack of a hydroxyl group on the carbon at the 6-position (C-6) and the L-configuration is the only levorotatory sugar utilized by mammalian systems.

Elevated levels of serum fucose have been reported in breast cancer, ovarian cancer, lung cancer, liver cancer, it plays a key role in cell metabolism and is an essential component of:
- EGFR of the Notch receptor, (mitosis regulator in hypoxic conditions);
- Lectins (extravasation);
- A, B, and H antigens from the surface of red blood cells
- Aquaporins, (hydration regulatory);
- Immunoglobulin;
- LEWIS Antigens;
- Mitochondria.

Serum level of L-fueuse was measured in several trials, L-fucose is essential in order to construct first, the malignant and then the metastatic phenotype of many human breast cancer. (PMCID: PMC3158734)
L-Fucose is essential in several key functions for the neoplastic progression, including: the development of metastasis, tumor invasion into the extracellular matrix and the activation of the Notch-Receptor. (Listinsky JJ, Siegal GP, Listinsky CMAM J Transl Rcs. 2011 Epub 2011 Jul 20).

The enzymatic assay of L-fucose free in the scrum .and in the urine (EC 1.1.1.122), is used as a marker for cancer. (Sakai, K Yamamoto, Clinical Chemistry 36: 474-476, 1990)
Aberrant glycosylation was identified in several tumor types. The expression of glycosylated epitopes specific for cancer represent a potential target for the diagnosis of cancer, (http Journal List Front Oneolv.4; 2014PMC3923139)
Recent and advanced approaches of glycomics aim to identify the fucosylation as a new tumor markers. Numerous studies support the functional significance of fucosylation in the various stages of carcinogenesis and tumor progression. (Kenta Moriwaki, Eiji Miyoshi- World J Hepatol 2010)

The glycans are also crucial in the development of metastases and thus the identification and monitoring of the latter can facilitate not only the diagnosis and prognosis, but also new preventive strategies (http: //www.ncbi.nlm .nih.gov / pubmed / 24592356).

### DISCLOSURE OF INVENTION

The dosage of serum glycoproteins fucosilate can be used as an effective indicator, associated with clinical procedures for the tumor diagnosis of. (RK Shetty, Bhandary SK, Kali A. J Clin Diagn Res. 2013 Dec; 7 (12): 2818-20. Doi: 10.7860 / JCDR / 2013 / 6681.3765. Epub 2013 Dec 15).

The dosage of total fucose (free or bound) presently done on the serum by various methods subsequent to a chemical or enzymatic deglycosylation.

You can dose L-fucose in serum, urine and sperm, using different methods, one of the first is the Winzler method, among the most recent: enzymatic methods, HPLC, gas chromatography, amperometric sensors and biosensors, lectins specific and other techniques. It must be emphasized that the fucose, free and bound to glycine or to Proteins (N or O-linked); the different types of bond are catalyzed by the same number of fucosyltransferase; to measure the total fucose in biological fluid it is necessary to make a deglycosylation.

The deglycosylation can be carried out by means an acidic hydrolysis (Winzler method) or by means of the deglycosylation kit, which follows the dosage of total fucose.

### SALIVARY TESTS BENEFITS

The test uses the reaction of molecular iodine with the methyl group of the fucose (free and bound; N-/ O-linked), because this functional group is not involved in the fucosylation reactions, and then remains available to form bonds with iodine, without prior deglycosylation

The salivary test therefore does not require the deglycosylation of residues of fucose, with a considerable saving of costs and reaction times, it is an indirect measure of the rate of total L-fucose (free or linked) by molecular iodine intake.

Saliva testing is performed for saliva, serum, urine or semen; It is a non-invasive test, fast, cheap this test can be associated to the various diagnostic prevention methods in use with of the significant saving in material and human labor.

The test can be associated to the normal chek up for the prevention of various diseases and in breast cancer, ovarian cancer, lung cancer, liver cancer, and after an exposure to risk factors (chemical and/or physical) but also for monitoring cancer progression, using invasive diagnostic and therapeutic techniques only if necessary condition.

### BRIEF DESCRIPTION OF DRAWINGS

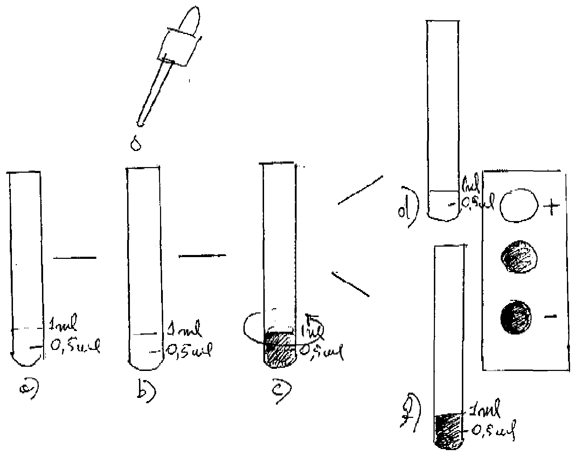

### DESCRIPTION OF THE FIGURE 1/1

The draw highlights sequence of steps:
a) place 1ml of saliva into a steril glass tube, graduated upward from tip to 1,0 ml in 0.5m divisions;
b) Place one drop of iodine solution at 5% to a sample of saliva;
c) Shake each test tube to mix the reagent and control the color changes every 30 seconds, for six observations in 3 minute;
d) The test is positive if the color change occurs (from brown to white) in a specific time interval (<1.5 minutes);
e) The test is negative if the color change does not occur in a specific time interval (>3.0 minutes);
f) compare the color change after 3 minutes of each sample and control with the colorimetric map.

### BEST MODE FOR CARRYING OUT THE INVENTION

Salivary diagnostic kit for early detection of cancer is based on the indirect dosage of L-fucose in saliva, using of a hydro-alcoholic solution of molecular iodine to 5%.

The increase of the salivary concentration of L-fucose might be associated with a tumor pathology.

The test is based on reaction between two apolar groups: the residue from methyl (CH3) of L-Fucose and the molecular iodine (I2).

The Fucose is a methyl pentose-amphiphilic sugar and the presence of the methyl apolar group determines its high affinity to the solvents or apolar compounds.

The fucose can be free in body fluids or bonded with N-glycosidic or O-glycosidic linkages in protein or glycoside groups.

These bonds are catalyzed by specific enzymes called Fucosyltrasferases.

The various links that the Fucose can form not involving the methyl group which is then available to interact with nonpolar substances.

The method comprises the following stages:
a) take a sample of saliva (1ml) in the morning without stimulation also by means of a saliva collector;
b) place 1ml of saliva into a steril glass tube, graduated upward from tip to 1,0 ml in 0.5m divisions;
c) Place one drop of iodine solution at 5% to a sample of saliva;
d) Shake each test tube to mix the reagent and control the color changes every 30 seconds, for six observations in 3 minute;
e) observe the color change of each sample and compare with the colorimetric map. Fig. 1(1)
f) The test is positive if the color change occurs (from brown to white) in a specific time interval (<1.5 minutes); (Fig.1, d)
h) The test is negative if the color change does not occur in a specific time interval (>3.0 minutes); (Fig.1, c)
i) If the color change is partial repeat the test after 20 days (Figure 1, d).

The color change in a specific time (<1.5 minutes), indicates an iodine absorption superior to a normal metabolism. The surplus of absorption is caused by reaction between iodine and L-Fucose free and bound to glycidic or protein residues.

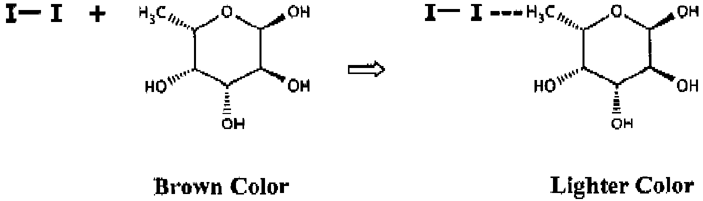

The iodine solution is brown, the link between Iodine and L-fucose determines the color change to a lighter color (Fig.1, d).

The test is positive if the color change occurs (from brown to white) in a specific time interval (<1.5 minutes); (Fig.1, d)
The kit comprises a colorimetric map of the color reference (Fig.1, f).

The absorption of the molecular iodine can be measured by spectrophotometric analysis.

The molecular iodine intake shown, by the color change, a significant increase of Fucose.

The method is characterized by the use of a hydro-alcoholic solution of molecular iodine to 5%, for the execution of the method according to any of the preceding claims, containing these components: 1 g KI, 4 g I2, 1ml Ethyl Alcohol, on 100ml of solution; this solution must be freshly prepared and stored at 4/5° C in the dark. Variables that can affect the test results:
They are the preanalytical variables that cancontribute to false-positive and false-negative results: diabetes, thyroid disease and related therapies, hormonal therapies, as well as high stress conditions.

### INDUSTRIAL APPLICABILITY

The salivary tests for the early detection of cancer, noninvasive, and easy-to-use screening method, economical can be used as mass screening for cancer prevention for any age group. There were no gender differences.

Prevention, so important today, could, with the help of the test, undergo a streamlining in the time and costs. The positive test does not detect any tumor localization, hut only provides the presence of metabolic alteration even in asymptomatic preclinical stages.

If the color change is partial or total, the test is repeated after two weeks, if the test result was confirmed again it is advisable to conduct further investigations in accordance with the practice, It is known, that analyzes based on the dosage of certain enzymes and / or some tumor antigens may give false positive or negative and that the fucosylation of these is important in prognosis. Again this helps to support the validity of the Fucose assay as a valid support for the prevention of cancer.

## Claims

1. **CLAIM 1** Method for the early detection of cancer, **characterized in that** 1ml of saliva reacts with a hydro-alcoholic solution of molecular iodine to 5%; this solution shows the increase salivary concentration of L-fucose, by means of the absorption of iodine; this method comprises the following steps:
a) take a sample of saliva (1ml) in the morning without stimulation also by means of a saliva collector;
b) place 1ml of saliva into a steril glass tube, graduated upward from tip to 1,0 ml in 0.5m divisions;
c) Place one drop of iodine solution at 5% to a sample of saliva;
d) Shake each test tube to mix the reagent and control the color changes every 30 seconds, for six observations in 3 minute;
e) observe the color change of each sample and control with the colorimetric map. Fig, 1(1)

2. Method, **AS CLAIMED IN CLAIM N1**, **characterized in that** the saliva sample (1ml) should be sampled in the morning without stimulation, also by means of a saliva collector; saliva must be analyzed within a few hours of sampling.

3. Method, **AS CLAIMED IN CLAIM N1**, **characterized in that** the saliva sample (1ml) should be placed in a sterile steril glass tube, graduated upward from tip to 1,0 ml in 0.5m divisions, each kit contains the colorimetric map.

4. Method, **AS CLAIMED IN CLAIM N1**, **characterized by** the use of a hydro-alcoholic solution of molecular iodine to 5%, for the execution of the method according to any of the preceding claims, containing these components: 1 g KI, 4 g 12, 1ml Ethyl Alcohol, on 100ml of solution; this solution must be freshly prepared and stored at 4/5° C in the dark.
